(19) Europäisches Patentamt
European Patent Office
Office européen des brevets

(11) EP 0 871 454 B1

(12) EUROPEAN PATENT SPECIFICATION

(45) Date of publication and mention of the grant of the patent: **12.11.2003 Bulletin 2003/46**

(21) Application number: **96923756.9**

(22) Date of filing: **12.07.1996**

(51) Int Cl.[7]: **A61K 31/66**, A61K 31/665, A61K 31/675, C07F 9/09, C07F 9/6574, C07K 5/06

(86) International application number: **PCT/US96/11625**

(87) International publication number: **WO 97/003679 (06.02.1997 Gazette 1997/07)**

# (54) PHOSPHOROUS-CONTAINING CYSTEINE AND SERINE PROTEASE INHIBITORS

PHOSPHOR ENTHALTENDE HEMMER VON CYSTEIN- UND SERIN-PROTEASEN

INHIBITEURS DE PROTEASES A CYSTEINE ET SERINE, CONTENANT DU PHOSPHORE

(84) Designated Contracting States:
**AT BE CH DE DK ES FI FR GB GR IE IT LI LU MC NL PT SE**

(30) Priority: **17.07.1995 US 1491 P**
**10.07.1996 US 679342**

(43) Date of publication of application: **21.10.1998 Bulletin 1998/43**

(73) Proprietor: **CEPHALON, INC.**
**West Chester, PA 19380 (US)**

(72) Inventors:
- **MALLAMO, John, P.**
  **Glenmore, PA 19343 (US)**
- **BIHOVSKY, Ron**
  **Wynnewood, PA 19096 (US)**
- **TAO, Ming**
  **Maple Glen, PA 19002 (US)**
- **WELLS, Gregory, J.**
  **West Chester, PA 19382 (US)**

(74) Representative: **Hallybone, Huw George et al**
**Carpmaels and Ransford,**
**43 Bloomsbury Square**
**London WC1A 2RA (GB)**

(56) References cited:
**EP-A- 0 644 197**

- **DOLLE R E ET AL: "Aspartyl.alpha.-((Diphenylphosphinyl)oxy) methyl Ketones as Novel Inhibitors of Interleukin-1.beta. Converting Enzyme. Utility of the Diphenylphosphinic Acid Leaving Group for the Inhibition of Cysteine Proteases" J. MED. CHEM. (JMCMAR,00222623);95; VOL.38 (2); PP.220-2, XP002075213 Sterling Winthrop Pharmaceuticals Research Division;Department of Medicinal Chemistry; Collegeville; 19426; PA; USA (US)**
- **OLEKSYSZYN J ET AL: "IRREVERSIBLE INHIBITION OF SERINE PROTEASES BY PEPTIDE DERIVATIVES OF (A-AMINOALKYL)PHOSPHONATE DIPHENYL ESTERS" BIOCHEMISTRY, vol. 30, no. 2, 15 January 1991, pages 485-493, XP000168882**
- **CHEMICAL ABSTRACTS, vol. 109, no. 23, 5 December 1988 Columbus, Ohio, US; abstract no. 211439, SCHLEMMER H ET AL: "Phosphorus-31 nuclear magnetic resonance spectroscopy of the phosphorylated tetrapeptide Gly-Gly-Asp-Ala" XP002075214 & MAGN. RESON. CHEM. (MRCHEG,07491581);88; VOL.26 (3); PP.260-3, Max-Planck Inst. Med. Res.;Dep. Biophys.; Heidelberg; Fed. Rep. Ger. (DE)**
- **CHEMICAL ABSTRACTS, vol. 100, no. 7, 13 February 1984 Columbus, Ohio, US; abstract no. 051966, RAMAGE R ET AL: "Application of phosphinic acids to peptide synthesis" XP002075215 & PEPT., PROC. EUR. PEPT. SYMP., 17TH (50GFAA);83; PP.157-62, UMIST;Dep. Chem.; Manchester; M60 1QD; UK (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

(Cont. next page)

- **DATABASE CAPLUS ON STN, Chemical Abstracts Service, (Columbus, Ohio, US), Accession No. 1988:611439, SCHLEMMER, H. et al., "Phosphorus-31 Nuclear Magnetic Resonance Spectroscopy of the Phosphorylated Tetrapeptide Gly-Gly-Asp-Ala"; & MAGN. RESON. CHEM. (1988), 26(3), 260-3 (Compound with Registry No. 117289-76-6).**
- **DATABASE CAPLUS ON STN, Chemical Abstracts Service, (Columbus, Ohio, US), Accession No. 1995:297974, DOLLE, R.E. et al., "Aspartylalpha-((Diphenylphosphinyl)oxy)methyl Ketones as Novel Inhibitors of Interleukin-1beta Converting Enzyme. Utility of the Diphenylphosphonic Acid Leaving Group for the Inhibition of Cysteine Proteases"; & J. MED. CHEM. (1995), 38(2), 220-2.**
- **DATABASE CAPLUS ON STN, Chemical Abstracts Service, (Columbus, Ohio, US), Accession No. 1995:867576, STERLING-WINTHROP, INC., "Preparation of Peptide Derivatives as Interleukin 1beta-Converting Enzyme Inhibitors"; & JP,A,07 025 887, 27 January 1995.**
- **DATABASE CAPLUS ON STN, Chemical Abstracts Service, (Columbus, Ohio, US), Accession No. 1984:51966, RAMAGE, R. et al., "Application of Phosphinic Acids to Peptide Synthesis"; & PEPT. PROC. EUR. PEPT. SYMP., 17th (1983), Meeting Date 1982, 157-62 (Compound with Registry No. 88577-10-0).**

## Description

## FIELD OF THE INVENTION

The present invention relates to inhibitors of cysteine or serine proteases, referred to herein as β-keto phosphates, methods for making these novel compounds, and methods for using the same are disclosed.

## BACKGROUND OF THE INVENTION

Numerous cysteine and serine proteases have been identified in human tissues. A "protease" is an enzyme which degrades proteins into smaller components (peptides). The terms "cysteine protease" and "serine protease" refer to proteases which are distinguished by the presence therein of a cysteine or serine residue which plays a critical role in the catalytic process. Mammalian systems, including humans, normally degrade and process proteins via a variety of enzymes including cysteine and serine proteases. However, when present at elevated levels or when abnormally activated, cysteine and serine proteases may be involved in pathophysiological processes.

For example, calcium-activated neutral proteases ("calpains") comprise a family of intracellular cysteine proteases which are ubiquitously expressed in mammalian tissues. Two major calpains have been identified; calpain I and calpain II. While calpain II is the predominant form in many tissues, calpain I is thought to be the predominant form in pathological conditions of nerve tissues. The calpain family of cysteine proteases has been implicated in many diseases and disorders, including neurodegeneration, stroke, Alzheimer's, amyotrophy, motor neuron damage, acute central nervous system injury, muscular dystrophy, bone resorption, platelet aggregation, cataracts and inflammation. Calpain I has been implicated in excitatory amino-acid induced neurotoxicity disorders including ischemia, hypoglycemia, Huntington's Disease, and epilepsy. The lysosomal cysteine protease cathepsin B has been implicated in the following disorders: arthritis, inflammation, myocardial infarction, tumor metastasis, and muscular dystrophy. Other lysosomal cysteine proteases include cathepsins C, H, L and S. Interleukin-1β converting enzyme ("ICE") is a cysteine protease which catalyzes the formation of interleukin-1β. Interleukin-1β is an immunoregulatory protein implicated in the following disorders: inflammation, diabetes, septic shock, rheumatoid arthritis, and Alzheimer's disease. ICE has also been linked to apoptotic cell death of neurons, which is implicated in a variety of neurodegenerative disorders including Parkinson's disease, ischemia, and amyotrophic lateral sclerosis (ALS).

Okeksyszyn, J. *et al*, Biochemistry vol. 30 No. 2 1991 pages 485-493 describes irreversible inhibition of serine proteases by peptide derivatives of (α-amino alkyl) phosphonate diphenyl esters.

Specific β-keto phosphinates have been described as inhibitors of ICE, cathepsin B, and calpain (R. E. Dolle, et al., J. Med. Chem. 1995 38, 220-222). *See also* European Patent Application Pub. No. 0 644 197 Al.

Cysteine proteases are also produced by various pathogens. The cysteine protease clostripain is produced by Clostridium histolyticum. Other proteases are produced by Trpanosoma cruzi, malaria parasites Plasmodium falciparum and P.vinckei and Streptococcus. Hepatitis A viral protease HAV C3 is a cysteine protease essential for processing of picornavirus structural proteins and enzymes.

Exemplary serine proteases implicated in degenerative disorders include thrombin, human leukocyte elastase, pancreatic elastase, chymase and cathepsin G. Specifically, thrombin is produced in the blood coagulation cascade, cleaves fibrinogen to form fibrin and activates Factor VIII; thrombin is implicated in thrombophlebitis, thrombosis and asthma. Human leukocyte elastase is implicated in tissue degenerative disorders such as rheumatoid arthritis, osteoarthritis, atherosclerosis, bronchitis, cystic fibrosis, and emphysema. Pancreatic elastase is implicated in pancreatitis. Chymase, an enzyme important in angiotensin synthesis, is implicated in hypertension, myocardial infarction, and coronary heart disease. Cathepsin G is implicated in abnormal connective tissue degradation, particularly in the lung.

Given the link between cysteine and serine proteases and various debilitating disorders, compounds which inhibit these proteases would be useful and would provide an advance in both research and clinical medicine. The present invention is directed to these, as well as other, important ends.

## SUMMARY OF THE INVENTION

The present invention is directed to novel cysteine and serine protease inhibitors referred to herein as β-keto phosphates. These novel compounds are represented by the following Formula I:

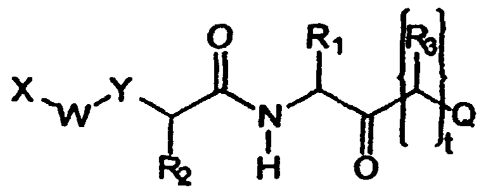

I

wherein: X, W, Y, $R_1$, $R_2$, $R_3$, t, J, Q, m, n, z, $R_4$ and $R_5$ are as set out in claim 1.

Some preferred embodiments of the compounds of Formula I are represented by compounds having the Formula Ia:

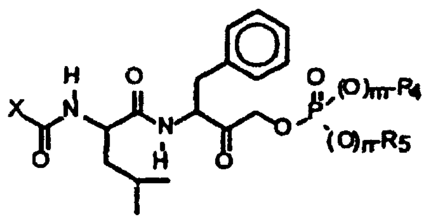

Ia

wherein X, $R_4$, $R_5$, m and n are as previously defined.

The compounds of the invention are useful for the irreversible inhibition of cysteine and serine proteases. Beneficially, these compounds find utility in a variety of settings. For example, in the research arena, the claimed compounds can be used, for example, in discovery of agents for treating disorders associated with abnormal and/or aberrant activity of cysteine and/or serine proteases. In a clinical arena, for example, the compounds can be used to alleviate, mediate, reduce, and/or prevent disorders which are associated with abnormal and/or aberrant activity of cysteine and/or serine proteases. Methodologies for making our β-keto phosphates are also disclosed.

These and other features of the compounds of the subject invention are set forth in more detail below.

**DETAILED DESCRIPTION**

Novel cysteine and serine protease inhibitors have been discovered which are represented by the general Formula I.

Preferred features of the invention are set out in claims 2 to 17.

In some preferred embodiments $R_1$ is aralkyl. In other preferred embodiments $R_2$ is alkyl. In further preferred embodiments $R_3$ is hydrogen. Preferably, Y is NH.

Also, in some preferred embodiments X is alkoxy, aralkyloxy, a carbohydrate moiety or, together with W, heteroalkylsulfonyl. In particularly preferred embodiments X is benzyloxy or *t*-butoxy.

In some preferred embodiments $R_4$ and $R_5$ are independently hydrogen, alkyl having 1 to 4 carbons optionally substituted with J where J is preferably alkyl or aryl, aryl substituted with J where J is preferably halogen or alkyl, aralkyl optionally substituted with J where J is preferably alkyl or aryloxy, or $R_4$ and $R_5$ taken together with the $-(O)_m-P(=O)-(O)_n-$ of Q form a six membered ring that is substituted by J.

In particularly preferred embodiments $R_4$ and $R_5$ are independently H, methyl, butyl, 2-ethylhexyl, 2-cyclohexylethyl, 2-phenylethyl, 4-chlorophenyl, benzyl, 2-methylbenzyl, and 3-phenoxybenzyl, or $R_4$ and $R_5$, taken together

with the the $-(O)_m-P(=O)-(O)_n-$ of Q form a six-membered ring having the formula:

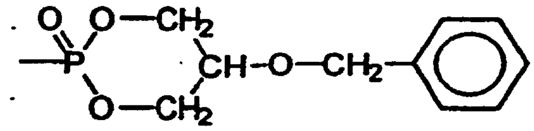

In some preferred embodiments, compounds of the invention have the Formula Ia:

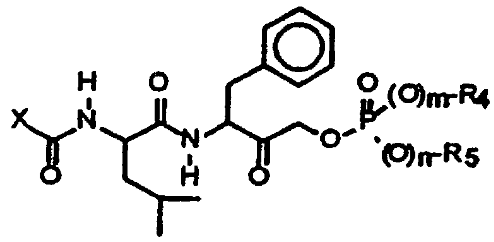

Ia

wherein X, $R_4$, $R_5$, m and n are as previously defined.

In some preferred embodiments of Formula Ia, $R_4$ and $R_5$ are independently hydrogen, methyl, butyl, benzyl, 2-ethylhexyl or 2-phenylethyl. In other preferred embodiments of Formula Ia, $R_4$ and $R_5$ are independently benzyl or 2-phenylethyl.

In other preferred embodiments of Formula Ia, X is benzyloxy or *t*-butoxy. In some preferred embodiments X, taken together with the carbonyl group of Formula Ia to which X is attached, is monoisopropylidine-2-keto-L-gulonyl or diisopropylidine-2-keto-L-gulonyl.

As used herein, the term "alkyl" is meant to include straight-chain, branched and cyclic hydrocarbon groups such as, for example, ethyl, isopropyl and cyclopropyl groups. Alkyl groups can contain one or two sites of unsaturation; i.e., carbon-carbon double or triple bonds. Preferred alkyl groups have 1 to about 10 carbon atoms. "Cycloalkyl" groups are cyclic alkyl groups. "Aryl" groups are aromatic cyclic compounds including but not limited to phenyl, tolyl, naphthyl, anthracyl, phenanthryl, pyrenyl, and xylyl. Preferred aryl groups include phenyl and naphthyl. The term "carbocyclic", as used herein, refers to cyclic groups in which the ring portion is composed solely of carbon atoms. The term "heterocyclic" refers to cyclic groups in which the ring portion includes at least one heteroatom such as O, N or S. "Heteroalkyl" groups are heterocycles containing solely single bonds within their ring portions, i.e. saturated heteroatomic ring systems. Heteroalkyl groups may contain sites of unsaturation outside their ring portions. Thus, for example, pyrrolidinonyl groups, which contain carbonyl carbon atoms within their ring systems, are heteroalkyl groups as defined herein. The term "lower alkyl" refers to alkyl groups of 1-4 carbon atoms. The term "halogen" refers to F, Cl, Br, and I atoms.

The term "aralkyl" denotes an alkyl group which is substituted with an aryl group, such as, for example, a benzyl group. Aralkyl groups can consist of two alkyl. groups bound to a single aryl group, such as groups having the formula:

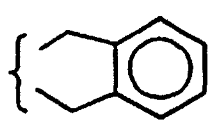

The term "aralkyloxy" denotes an aralkyl group attached through an oxygen atom. The term "heteroaryl" denotes aryl groups having one or more heteroatoms contained within an aryl ring. "Heteroaralkyl" groups are aralkyl groups which

have one or more heteroatoms in their aryl ring portion. The term "carbohydrate" includes monosaccharides, disaccharides, and polysaccharides, as well as their protected derivatives, such as, for example, mono- and diisopropylidine derivatives.

Because the β-keto phosphates of the invention inhibit cysteine proteases and serine proteases, they can be used in both research and therapeutic settings.

In a research environment, preferred compounds having defined attributes can be used to screen for natural and synthetic compounds which evidence similar characteristics in inhibiting protease activity. Inhibition of cysteine protease or serine protease activity can be measured by determining the rate of inactivation of a protease using a compound of the invention. The compounds can also be used in the refinement of *in vitro* and *in vivo* models for determining the effects of inhibition of particular proteases on particular cell types or biological conditions. In a therapeutic setting, given the connection between cysteine proteases and certain defined disorders, and serine proteases and certain defined disorders, compounds of the invention can be utilized to alleviate, mediate, reduce and/or prevent disorders which are associated with abnormal and/or aberrant activity of cysteine proteases and/or serine proteases.

In preferred embodiments, compositions are provided for inhibiting a serine protease or a cysteine protease comprising a compound of the invention. In other preferred embodiments, methods in vitro are provided for inhibiting serine proteases or cysteine proteases comprising contacting a protease selected from the group consisting of serine proteases and cysteine proteases with an inhibitory amount of a compound of the invention.

The disclosed compounds of the invention are useful for the irreversible inhibition of cysteine proteases and serine proteases. As used herein, the terms "inhibit" and "inhibition" mean having an adverse effect on enzymatic activity. The term "irreversible," when used to modify "inhibit" and "inhibition" means that such adverse effect on catalytic activity can not be readily reversed. An inhibitory amount is an amount of a compound of the invention effective to inhibit a cysteine and/or serine protease.

Pharmaceutically acceptable salts of the cysteine and serine protease inhibitors also fall within the scope of the compounds as disclosed herein. The term "pharmaceutically acceptable salts" as used herein means an inorganic acid addition salt such as hydrochloride, sulfate, and phosphate, or an organic acid addition salt such as acetate, maleate, fumarate, tartrate, and citrate. Examples of pharmaceutically acceptable metal salts are alkali metal salts such as sodium salt and potassium salt, alkaline'earth metal salts such as magnesium salt and calcium salt, aluminum salt, and zinc salt. Examples of pharmaceutically acceptable ammonium salts are ammonium salt and tetramethylammonium salt. Examples of pharmaceutically acceptable organic amine addition salts are salts with morpholine and piperidine. Examples of pharmaceutically acceptable amino acid addition salts are salts with lysine, glycine, and phenylalanine.

Compounds provided herein can be formulated into pharmaceutical compositions by admixture with pharmaceutically acceptable nontoxic excipients and carriers. As noted above, such compositions may be prepared for use in parenteral administration, particularly in the form of liquid solutions or suspensions; or oral administration, particularly in the form of tablets or capsules; or intranasally, particularly in the form of powders, nasal drops, or aerosols; or dermally, via, for example, transdermal patches; or prepared in other suitable fashions for these and other forms of administration as will be apparent to those skilled in the art.

The composition may conveniently be administered in unit dosage form and may be prepared by any of the methods well known in the pharmaceutical art, for example, as described in *Remington's Pharmaceutical Sciences* (Mack Pub. Co., Easton, PA, 1980). Formulations for parenteral administration may contain as common excipients sterile water or saline, polyalkylene glycols such as polyethylene glycol, oils and vegetable origin, hydrogenated naphthalenes and the like. In particular, biocompatible, biodegradable lactide polymer, lactide/glycolide copolymer, or polyoxyethylene-polyoxypropylene copolymers may be useful excipients to control the release of the active compounds. Other potentially useful parenteral delivery systems for these active compounds include ethylene-vinyl acetate copolymer particles, osmotic pumps, implantable infusion systems, and liposomes. Formulations for inhalation administration contain as excipients, for example, lactose, or may be aqueous solutions containing, for example, polyoxyethylene-9-lauryl ether, glycocholate and deoxycholate, or oily solutions for administration in the form of nasal drops, or as a gel to be applied intranasally. Formulations for parenteral administration may also include glycocholate for buccal administration, a salicylate for rectal administration, or citric acid for vaginal administration. Formulations for transdermal patches are preferably lipophilic emulsions.

The materials of this invention can be employed as the sole active agent in a pharmaceutical or can be used in combination with other active ingredients, e.g., other growth factors which could facilitate neuronal survival or axonal regeneration in diseases or disorders.

The concentrations of the compounds described herein in a therapeutic composition will vary depending upon a number of factors, including the dosage of the drug to be administered, the chemical characteristics (e.g., hydrophobicity) of the compounds employed, and the route of administration. In general terms, the compounds of this invention may be provided in effective inhibitory amounts in an aqueous physiological buffer solution containing about 0.1 to 10% w/v compound for parenteral administration. Typical dose ranges are from about 1 μg/kg to about 1 g/kg of body

weight per day; a preferred dose range is from about 0.01 mg/kg to 100 mg/kg of body weight per day. Such formulations typically provide inhibitory amounts of the compound of the invention. The preferred dosage of drug to be administered is likely, however, to depend on such variables as the type and extent of progression of the disease or disorder, the overall health status of the particular patient, the relative biological efficacy of the compound selected, and formulation of the compound excipient, and its route of administration.

As used herein, the term "contacting" means directly or indirectly causing at least two moieties to come into physical association with each other. Contacting thus includes physical acts such as placing the moieties together in a container, or administering moieties to a patient. Thus, for example administering a compound of the invention to a human patient evidencing a disease or disorder associated with abnormal and/or aberrant activity of such proteases falls within the scope of the definition of the term "contacting".

The invention is further illustrated by way of the following examples which are intended to elucidate the invention. These examples are not intended, nor are they to be construed, as limiting the scope of the disclosure.

## Examples

Compounds of the invention were prepared by the following procedures.

### Starting Materials:

Phenylalanine chloromethylketone can be purchased from various commercial sources (e.g., BACHEM Bioscience, Inc.) and was used as received. Benzyloxycarbonyl and t-butoxycarbonyl protected dipeptide bromomethyl ketones were prepared from the corresponding diazomethylketones by treatment with HBr/AcOH or HBr (gas) according to the standard procedures cited and described in Harbeson, S. L. et al., *J. Med. Chem.* **1989,** *32,* 1378-1392. (Morpholinylsulfonyl)-L-leucine was prepared according to Repine's procedure (Repine, J.T. et al., *J. Med. Chem.,* **1992** *35,* 1032-1042). *N*-terminal protected dipeptide chloromethyl ketones were prepared from their corresponding *N*-terminal protected-L-leucine and phenylalanine chloromethylketone under isobutyl chloroformate mediated coupling conditions (Rich D. L. et al., *J. Med. Chem.* **1992** *35,* 3802-3812). *N*-t-Butoxycarbonyl-L-leucinal was prepared from t-butoxycarbonyl-L-leucine (Goel, O. P. et al., *Org. Syn.* **1993,** *Coll. Vol. VIII*, 68).

### Analyses:

FAB mass spectra were obtained by M-Scan, Inc. Ion spray mass spectra were determined with Fisons VG platform mass spectrometer.

### Example 1: Intermediate

**Benzyloxycarbonyl-L-leucyl-L-phenylalanyl bromomethyl ketone** (m.p. 135.5-136.5°C) was prepared by the procedure described by Harbeson et al., *supra.*

### Example 2: Intermediate

***t*-Butoxycarbonyl-L-leucyl-L-phenylalanyl bromomethyl ketone** (m.p. 120-122°C) was prepared by the procedure described by Harbeson et al., *supra.*

### Example 3: Intermediate

**Diisopropylidine-2-keto-L-gulonyl-L-leucyl-L-phenylalanyl** chloromethyl ketone (m.p. 74-75°C) was prepared by a modification of the procedure described by Rich et al., *supra*.

### Example 4: Intermediate

**Diisopropylidine-2-keto-L-gulonyl-L-leucyl-L-phenylalanyl iodomethyl ketone** was prepared from diisopropylidine-2-keto-L-gulonyl-L-leucyl-L-phenylalanyl chloromethyl ketone with 1.5 eq. of NaI in acetone and used for the next step without purification.

### Example 5: Intermediate

**Morpholinylsulfonyl-L-leucyl-L-phenylalanyl choloromethyl ketone** (m.p.145-146.5°C) was prepared by

a modification of the procedure described by Rich et al., *supra.*

**Example 6: Intermediate**

**Morpholinylsulfonyl-L-leucyl-L-phenylalanyl iodomethyl** ketone was prepared from morpholinylsulfonyl-L-leucyl-L-phenylalanyl chloromethyl ketone with 1.5 eq. of NaI in acetone and used for the next step without purification.

**Example 7: Intermediate**

**Diphenethyl phosphate:**

A solution of 0.79g (4.0 mmol) of *N,N*-diisopropyl methylphosphonamidic chloride in 0.6 mL of $CH_2Cl_2$ was stirred at 0°C under $N_2$ as a mixture of phenethyl alcohol (0.50g, 4.0 mmol) and pyridine (0.32g, 1.0eq.) in 0.6 mL of $CH_2Cl_2$ was added. The solution was warmed to room temperature and stirred overnight (~16h). The solution was then cooled to 0°C and 0.8 mL of MeOH and 2.4 mL of 30% $H_2O_2$ were slowly added. The mixture was warmed to room temperature and stirred for 5 hours, and the product was extracted with $CH_2Cl_2$ (2 X 10mL). The combined organic layers were washed with 10% $Na_2SO_3$ (10 mL), 1N HCl (10 mL), brine (10 mL), dried over magnesium sulfate and concentrated. Flash, chromatography (50% ethyl acetate in hexane) gave 0.51 (39%) of the bis(phenethyl) methyl phosphate as a clear oil.

A solution of 0.26g (0.8 mmol) of bis(phenethyl) methyl phosphate in 3.2 mL of dry acetonitrile was stirred at 0°C under $N_2$ while 0.23 mL (2.18 eq.) of TMSBr was added dropwise. The solution was stirred at 0°C for 1.5 hours. The solvent was removed and the residue was dissolved in NaOH (1.0 eq.) in MeOH solution. After 30 minutes at room temperature, the solution was concentrated, and the white solid was taken up in ether (6.0 mL) and filtered to give sodium bis(phenethyl) phosphate.

A solution of sodium bis(phenethyl) phosphate (0.24 mg, 0.73mmol) in 1.0 mL of water was stirred as 2.0 mL of concentrated hydrochloric acid (2.0 mL) was added. The organic layer was extracted with $CH_2Cl_2$ (3X6mL) and dried over magnesium sulfate. Concentration gave 0.18 g (81%) of bis(phenethyl) phosphate. MS: 305 m/z (M-1).

**Example 8: Intermediate**

**Bis(2-cyclohexylethyl) phosphate** was prepared according to the general procedure given for bis(phenethyl) phosphate in Example 7. MS: 317 m/z (M-1).

**Example 9: Intermediate**

**Bis(3-phenoxybenzyl) phosphate:**

A solution of 0.79 ml (4.0 mmol) of *N,N*-diisopropyl methylphosphonamidic chloride in 0.6 mL of $CH_2Cl_2$ was stirred at 0°C under $N_2$ as a mixture of 3-phenoxybenzyl alcohol (1.6g, 8.0 mmol) and pyridine (0.32g, 1.0eq.) in 0.6 mL of $CH_2Cl_2$ was added. The solution was warmed to room temperature and stirred for 5 hours. The solution was then cooled to 0°C and 0.8 mL of MeOH and 2.4 mL of 30%$H_2O_2$ were slowly added. The mixture was warmed to room temperature and stirred for 5 hours. The product was extracted with $CH_2Cl_2$ (2 X 10mL). The combined organic layers were washed with 10% $Na_2SO_3$ (10 mL), 1N HCl (10 mL), brine (10 mL), dried over magnesium sulfate and concentrated. Flash chromatography (50% ethyl acetate in hexane) gave 0.51g (39%) of the bis(3-phenoxybenzyl) methyl phosphate as a clear oil.

A solution of 0.48g (1.0 mmol) of bis(3-phenoxybenzyl) methyl phosphate in 10 mL of toluene was stirred at rt under $N_2$ while 0.125g (1.1 eq.) of 1,4-diazabicyclo[2.2.2]octane was added. The solution was refluxed for 4.0 hours. The solvent was removed and residue was diluted in 5% HCl solution (10 mL), extracted with EtOAc (3X10mL) and dried over magnesium sulfate. Concentration gave 0.33 g (72%) of bis(3-phenoxybenzyl) phosphate. MS: 461 m/z (M-1).

**Example 1: Intermediate**

**Bis(2-methylbenzyl) phosphate** was prepared according to the general procedure given for of bis(3-phenoxybenzyl)phosphate in Example 9. MS: 305 m/z (M-1).

**Example 2: Intermediate**

**5-Benzyloxy-2-hydroxy-1,3,2-dioxaphosphorinane 2-oxide**

A solution 0.5g (2.75 mmol) of 2-benzyloxy-1,3-propanediol in 3.0 mL of pyridine was stirred at 0 °C under $N_2$ as 0.37g (2.5 mmol) of methyl dichlorophosphate was added dropwise over 15 mins to keep the solution under 10 °C. The cold bath was removed and the reaction mixture was stirred overnight at 20 °C(~14h). The mixture was filtered, and washed with benzene (10 mL), and the filtrate was evaporated. The residue was dissolved in benzene : $CH_2Cl_2$ (1:1, 20 mL), washed with $H_2O$ (10 mL), saturated $NaHCO_3$ (10 mL), saturated NaCl (10 mL) and dried over magnesium sulfate. Evaporation afforded 0.26 g (40%) of 5-benzyloxy-2-methyl-1,3,2-dioxaphosphorinane-2-oxide.

A solution of 0.19 g (0.75 mmol) of (2-benzyloxy)propylene methyl phosphate in 7.5 mL of toluene was stirred at rt under $N_2$ while 0.093g (1.1 eq.) of 1,4-diazabicyclo[2.2.2]octane was added. The solution was refluxed for 4.0 hours. The solvent was removed and the residue was diluted in 5% HCl solution (10 mL), extracted with EtOAc (3X10mL) and dried over magnesium sulfate. Concentration gave 0.14g (74%) of 5-benzyloxy-2-hydroxy-1,3,2-dioxaphosphorinane-2-oxide. MS: 243 m/z (M-1).

**Example 3: Methods for Preparing Inhibitors**

Methods A and B, are representative methods for preparing compounds of the invention.

**Method A:** To a solution of the appropriate bromo or iodoketone (0.1-0.2 mmol) in 1.0-2.0 mL of DMF was added anhydrous potassium fluoride (3.5 eq.) under $N_2$. After the mixture was stirred at room temperature for 5 minutes, a phosphate, phosphonate, phosphinic acid, or phosphonamidate (1.2 eq.) was added, and the mixture was stirred for 3-72 hours. The reaction mixture was diluted with $CH_2Cl_2$ and filtered through celite. The solution was washed with water, 5% $NaHCO_3$ solution, 5% aqueous citric acid, brine and dried over magnesium sulfate. Purification by flash chromatography or crystallization afforded the desired product.

**Method B:** A solution of the appropriate bromo or iodoketone (0.1-0.2 mmol) in 0.5-1.0 mL of $CH_2Cl_2$ was stirred at 0°C under Ar while diisopropylethylamine (3.3 eq.) was added dropwise by syringe. After 5 minutes, a phosphate or phosphinic acid (1.2 eq.) was added, and the reaction was warmed to room temperature and stirred for 3-24 hours. The reaction was diluted with ethyl acetate and washed with 5% $NaHCO_3$ solution, 5% aqueous citric acid, brine and dried over $MgSO_4$. Purification by flash chromatography or crystallization afforded the desired product. The following Examples list method, starting materials, reaction times, purification methods, yields, physical properties, elemental analyses and/or mass spectra.

**Example 4:**

**Dibenzyl (3*S*)-[3-[(N-Benzyloxycarbonyl-L-leucyl)amino]-2-oxo-4-phenylbutyl] phosphate:**

Method A; Cbz-L-leucyl-L-phenylalanyl bromomethyl ketone; dibenzyl phosphate (Aldrich Chemical Co.); 24h; flash chromatography (30% ethyl acetate in hexane); yield 44%; mp 117-118°C; FABMS: 687 m/z (M+H).

Analysis calculated for $C_{38}H_{43}N_2O_8P$: C, 66.46; H, 6.31; N, 4.08. Found: C, 66.18; H, 5.94; N, 4.41.

**Example 5:**

**Dibenzyl (3*S*)-[3-[(N-t-Butoxycarbonyl-L-leucyl)amino]-2-oxo-4-phenylbutyl]phosphate:**

Method A; Boc-L-leucyl-L-phenylalanyl bromomethyl ketone; dibenzyl phosphate; 17 hours; flash chromatography (2:1 ethyl acetate : hexane); yield 46%; mp 84-85°C; MS: 653 m/z (M+H); 675 m/z (M+Na).

Analysis calculated for $C_{35}H_{45}N_2O_8P$: C, 64.40; H, 6.95; N, 4.29. Found: C, 64.32; H, 7.10; N, 4.75.

**Example 6:**

**Dibenzyl (3*S*)-[3- [(N-morpholinylsulfonyl-L-leucyl)amino]-2-oxo-4-phenylbutyl] phosphate:**

Method A; morpholinylsulfonyl-L-leucyl-L-phenylalanyl iodomethyl ketone; dibenzyl phosphate; 24 hours; flash chromatography (50% ethyl acetate in hexane); yield 38%; mp 52-52.5°C; MS: 702 m/z (M+H); 724 m/z (M+Na).

Analysis calculated for $C_{34}H_{44}N_3O_9PS$: C, 58.19; H, 6.32; N, 5.99. Found: C, 57.25; H, 6.25; N, 6.31.

**Example 7:**

**Dibenzyl (3*S*)-[3-[(N-diisopropylidine-2-keto-L-gulonyl-L-leucyl)amino]-2-oxo-4-phenylbutyl] phosphate:**

Method A; Diisopropylidine-2-keto-L-gulonyl-L-leucyl-L-phenylalanyl iodomethyl ketone; dibenzyl phosphate; 20 hours; flash chromatography (50% ethyl acetate in hexane); yield 41%; mp 61-62°C; MS: 809 m/z (M+H); 831 m/z (M+Na).

Analysis calculated for $C_{42}H_{53}N_2O_{12}P.0.7\ H_2O$: C, 61.40; H, 6.67; N, 3.41. Found: C, 61.26; H, 6.56; N, 3.30.

**Example 8:**

**Dibenzyl (3*S*)-[3-[(N-monoisopropylidine-2-keto-L-gulonyl-L-leucyl)amino]-2-oxo-4-phenylbutyl] phosphate):**

A solution of 12.1 mg (0.015mmol) of dibenzyl (3*S*)-[3-[(N-diisopropylidine-2-keto-L-gulonyl-L-leucyl)amino]-2-oxo-4-phenylbutyl] phosphate in 0.5 mL of THF was stirred at room temperature as 0.5 mL of 1.0 N HCl was added. The mixture was stirred at room temperature for 4 hours. The solution was diluted with 15 mL of $CH_2Cl_2$, washed with water (2x5mL), brine (5 mL), dried over magnesium sulfate. Concentration of the solution gave 8.1 mg (70%) of the product. MS: 769 m/z (M+H); 791 m/z (M+Na).

**Example 9:**

**Bis(2-methylbenzyl) (3*S*)-[3-[(N-Benzyloxycarbonyl-L-leucyl)amino]-2-oxo-4-phenylbutyl] phosphate:**

Method A; Cbz-L-leucyl-L-phenylalanyl bromomethyl ketone; bis(2-methylbenzyl) phosphate; 24h; flash chromatography (50% ethyl acetate in hexane); yield 15%; mp 87.5-88.5 °C; MS: 737 m/z (M+Na).

**Example 10:**

**Bis(3-phenoxybenzyl) (3*S*)-[3-[(N-Benzyloxycarbonyl-L-leucyl)amino]-2-oxo-4-phenylbutyl] phosphate:**

Method A; Cbz-L-leucyl-L-phenylalanyl bromomethyl ketone; bis(3-phenoxybenzyl) phosphate; 20h; flash chromatography (50% ethyl acetate in hexane); yield 20%; mp 71.5-73 °C; MS: 871 m/z (M+H); 893 m/z (m+Na).

**Example 11:**

**Dihydrogen (3*S*)-[3-[(N-t-Butoxycarbonyl-L-leucyl)amino]-2-oxo-4-phenylbutyl] phosphate:**

A suspension of 30 mg of 20% $Pd(OH)_2$ in a solution of dibenzyl (3*S*)-[3-[(N-t-Butoxycarbonyl-L-leucyl)amino]-2-oxo-4-phenylbutyl] phosphate (30 mg, 0.046 mmol) in 1.0 mL of ethyl acetate was stirred under $H_2$ (1 atm) at room temperature for 4 hours. The catalyst was filtered through Celite™ and the filtrate was concentrated to give the crude product. Flash chromatography (10% MeOH in $CH_2Cl_2$) gave 8 mg of pure product. MS: 471 m/z (M-1).

**Example 12:**

**Dimethyl (3*S*)-[3-[(N-Benzyloxycarbonyl-L-leucyl)amino]-2-oxo-4-phenylbutyl] phosphate:**

Method B; Cbz-L-leucyl-L-phenylalanyl bromomethyl ketone; dimethyl phosphate (Pfaltz & Bauer Inc.); 24 hours; flash chromatography (50% ethyl acetate in hexane); yield 29%; mp 87-88.5°C; MS: 535 m/z (M+H); 557 m/z (M+Na).

**Example 13:**

**Dibutyl (3*S*)-[3-[(N-Benzyloxycarbonyl-L-leucyl)amino]-2-oxo-4-phenylbutyl] phosphate:**

Method A; Cbz-L-leucyl-L-phenylalanyl bromomethyl ketone; dibutyl phosphate (Fluka Chemical Co.); KF/AlO3 (substituted for KF, 40% Aldrich Chemical Co.); 30h; flash chromatography (30% ethyl acetate in hexane) and recrystallization from ether/petroleum ether; yield 35%; mp 76-77°C; MS: 619 m/z (M+H); 641 m/z (M+Na).

Analysis calculated for $C_{32}H_{47}N_2O_8P$: C, 62.12; H, 7.66; N, 4.53. Found: C, 62.21; H, 7.64; N, 4.48.

**Example 14:**

**Bis(2-ethylhexyl) (3*S*)-[3-[(N-Benzyloxycarbonyl-L-leucyl)amino]-2-oxo-4-phenylbutyl] phosphate:**

Method A; Cbz-L-leucyl-L-phenylalanyl bromomethyl ketone; bis(2-ethylhexyl) phosphate (Aldrich Chemical Co.); 24h; flash chromatography (30% ethyl acetate in hexane); yield 25%; mp 88-89.5°C; FABMS: 731 m/z (M+H).

Analysis calculated for $C_{40}H_{63}N_2O_8P$: C, 65.73; H, 8.69; N, 3.83. Found: C, 65.64; H, 8.42; N, 3.96.

**Example 15:**

**Bis (2-cyclohexylethyl) (3*S*)-[3-[(N-t-Butoxycarbonyl-L-leucyl)amino]-2-oxo-4-phenylbutyl] phosphate:**

Method A; Cbz-L-leucyl-L-phenylalanyl bromomethyl ketone; bis(2-cyclohexylethyl) phosphate; 24h; flash chromatography (50% ethyl acetate in hexane); yield 13%; mp 51-52.5 °C; FABMS: 715 m/z (M+Na).

**Example 16:**

**Diphenethyl (3*S*)-[3-[(N-Benzyloxycarbonyl-L-leucyl)amino]-2-oxo-4-phenylbutyl] phosphate:**

Method A; Cbz-L-leucyl-L-phenylalanyl bromomethyl ketone; bis(phenethyl) phosphate; 24 hours; flash chromatography (50% ethyl acetate in hexane); yield 62%; m.p. 89-90°C; MS: 715 m/z (M+H); 737 m/z (M+Na).

Analysis calculated for $C_{40}H_{47}N_2O_8P$: C, 67.20; H, 6.63; N, 3.92. Found: C, 67.04; H, 6.64; N, 3.85.

**Example 17:**

**1-[(3*S*)-3-[(N-Benzyloxycarbonyl-L-leucyl)amino]-2-oxo-4-phenylbutyl]oxy]-5-benzyloxy-1,3,2-dioxaphosphorinane 2-oxide**

Method A; Cbz-L-leucyl-L-phenylalanyl bromomethyl ketone; 5-benzyloxy-2-hydroxy-1,3,2-dioxaphosphorinane 2-oxide; 16 hours; crystallization from ethyl acetate in hexane; yield 10%; m.p. 150-151°C; MS: 675 m/z (M+Na).

**Example 18:**

**Inhibition and Rate of Inactivation of Cysteine Protease Activity**

To evaluate inhibitory activity, stock solutions (40 times concentrated) of each compound to be tested were prepared in 100% anhydrous DMSO and 5 µl of each inhibitor preparation were aliquoted into each of three wells of a 96 well plate. Calpain I, prepared by a modification of the method of W. J. Lee et al. (Biochem. Internatl. 22: 163-171 (1990)), was diluted into assay buffer (i.e., 50mM Tris, 50mM NaCl, 1mM EDTA, 1mM EGTA, and 5mM β-mercaptoethanol, pH 7.5 including 0.2mM Succ-Leu-Tyr-MNA) and 175 µl aliquoted into the same wells containing the independent inhibitor stocks as well as to positive control wells containing 5 µl DMSO, but no compound. To start the reaction, 20 µl of 50 mM $CaCl_2$ in assay buffer was added to all wells of the plate, excepting three, which were used as background signal baseline controls. Substrate hydrolysis was monitored every 5 minutes for a total of 30 minutes. Substrate hydrolysis in the absence of inhibitor was linear for up to 15 minutes.

Inhibition of calpain I activity was calculated as the percent decrease in the rate of substrate hydrolysis in the presence of inhibitor ($v_i$) relative to the rate in its absence ($v_o$). Comparison between $v_o$ and $v_i$ was made within the linear range for substrate hydrolysis. For screening, compounds were tested at 10 µM and 0.1 µM. Compounds having 50% inhibition at 10 µM were considered active. Apparent second order rate constants were determined from analysis of reaction progress curves under pseudo-first order conditions. Each determination represents the mean of three or more independent single cuvette analyses continually monitored via a Perkin-Elmer LS50B spectrofluorimeter. The rate of inhibition of hydrolysis was obtained by fitting the curve to the exponential equation (1):

$$y = Ae^{-(kobs\ -\ t)} + B \qquad (1)$$

In the above equation 1, y is $P_t$, which is the amount of product formed at time t; $k_{obs}$ is the pseudo-first order rate

constant for inactivation; A is a constant which is the amplitude of the reaction, given by $[P_o-P_\infty]$, which is the difference between the product formed at t=0 ($P_o$) and the maximal product formed when the reaction is complete ($P_\infty$); B is a constant which is the maximal product formed when the reaction is complete ($P_\infty$); $k_{app}$ is the apparent second order rate constant, determined as $k_{obs}/[I]$, where [I] is inhibitor concentration. $k_{app}$ was corrected for the presence of substrate to give the second order rate constant $k_2$ according to equation (2):

$$k_2 = k_{app} (1 + [S] / K_m) \quad (2)$$

wherein [S] is substrate concentration, and $K_m$ is the Michaelis constant.

Values for $k_{obs}/I$ are given in Table I for compounds tested at 0.1 μM.

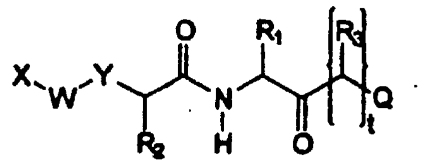

Table I

| Ex. # | X-W-Y- | Q | t | $k_{obs}/I \times 10^{-3}\ M^{-1}s^{-1}$ | % Inhib. @ 0.1 uM |
|---|---|---|---|---|---|
| | $R_1 = CH_2Ph$; $R_2$ = iBu; $R_3$ = H | | | | |
| 34 | $C_6H_5CH_2OCONH$ | $OP(O)(OCH_2C_6H_5)_2$ | 1 | 100 | |
| 35 | $t\text{-}C_4H_9OCONH$ | $OP(O)(OCH_2C_6H_5)_2$ | 1 | 242 | 100 |
| 36 | Morpholinyl sulfonyl-NH | $OP(O)(OCH_2C_6H_5)_2$ | 1 | 182 | |
| 37 | Diisopropylidine-2-keto-L-gulonyl-NH | $OP(O)(OCH_2C_6H_5)_2$ | 1 | 113 | |
| 38 . | Monoisopropylidine-2-keto-L-gulonyl-NH | $OP(O)(OCH_2C_6H_5)_2$ | 1 | 116 | |
| 39 | $C_6H_5CH_2OCONH$ | $OP(O)(OCH_2C_6H_4\text{-}2\text{-}CH_3)_2$ | 1 | 365 | |
| 40 | $C_6H_5CH_2OCONH$ | $OP(O)(OCH_2C_6H_4\text{-}3\text{-}OC_6H_5)_2$ | 1 | | 53 |
| 41 | $t\text{-}C_4H_9OCONH$ | $OP(O)(OH)_2$ | 1 | <20 | 7 |
| 42 | $C_6H_5CH_2OCONH$ | $OP(O)(OCH_3)_2$ | 1 | 20 | |
| 43 | $C_6H_5CH_2OCONH$ | $OP(O)(OC_4H_9)_2$ | 1 | 40 | |
| 44 | $C_6H_5CH_2OCONH$ | $OP(O)(OCH_2CH(C_2H_9)C_4H_9)_2$ | 1 | 1 | |
| 45 | $t\text{-}C_4H_9OCONH$ | $OP(O)(OCH_2CH_2\text{-}cyclohexyl)_2$ | 1 | | 28 |
| 46 | $C_6H_5CH_2OCONH$ | $OP(O)(OCH_2CH_2C_4H_9)_2$ | 1 | 380 | 99 |
| 47 | $C_6H_5CH_2OCONH$ | $OP(O)(OCH_2)_2CHOCH_2C_6H_5$ | 1 | | 47 |

## Claims

1. A compound of the formula:

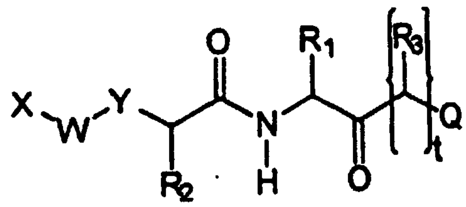

wherein:

X is selected from the group consisting of aryl having from 6 to 14 carbons; heteroaryl having from 6 to 14 ring atoms; aralkyl having from 7 to 15 carbons; alkyl having from 1 to 10 carbons, alkenyl and alkynyl each having up to 10 carbons and containing one or two sites of unsaturation, cycloalkyl having up to 10 carbon atoms, said alkyl, alkenyl, alkynyl and cycloalkyl groups being optionally substituted with one or more J groups; heterocycloalkyl having from 2 to 7 carbons; alkoxy having from 1 to 10 carbons; aralkyloxy having from 7 to 15 carbons; and a carbohydrate moiety optionally containing one or more alkylated hydroxyl groups;
W is selected from the group consisting of carbonyl and $SO_2$;
Y is selected from the group consisting of NH and $(CH_2)_k$ where k is an integer from 0 to 3;
$R_1$ and $R_2$ are independently selected from the group consisting of hydrogen; alkyl having from 1 to 14 carbons, alkenyl and alkynyl each having up to 10 carbon atoms and containing one or two sites of unsaturation, and cycloalkyl having from 3 to 10 carbons, said alkyl, alkenyl, alkynyl and cycloalkyl groups being optionally substituted with one or more J groups;
$R_3$ is selected from the group consisting of hydrogen, alkyl having from 1 to 4 carbon atoms, alkenyl and alkynyl each having up to 4 carbon atoms and containing one or two sites of unsaturation, cycloalkyl having up to 4 carbon atoms; aryl; heteroaryl; aralkyl; and heteroaralkyl;
t is 1;
J is selected from the group consisting of halogen; alkyl having 1 to 10 carbons, alkenyl and alkynyl each having up to 10 carbons and one or two sites of unsaturation, cycloalkyl having up to 10 carbons; aryl; heteroaryl; amino optionally substituted with one to three of any of aryl, alkyl having 1 to 4 carbons, alkenyl or alkynyl each having up to 4 carbons and one or two sites of unsaturation and cycloalkyl having up to 4 carbons; guanidino; alkoxycarbonyl; alkoxy; hydroxy; aryloxy; aralkyloxy; heterocycloalkyl; and carboxy; and
Q has the formula

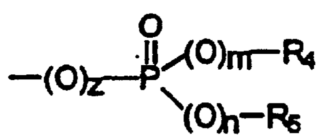

wherein:

m, n and z are each 1;
$R_4$ and $R_5$ are each independently selected from the group consisting of hydrogen; alkyl having 1 to 4 carbons, alkenyl and alkynyl each having up to 4 carbons and one or two sites of unsaturation, cycloalkyl having up to 4 carbons, said alkyl, alkenyl, alkynyl and cycloalkyl groups optionally substituted with J; aryl optionally substituted with J; aralkyl optionally substituted with J; and heteroaryl optionally substituted with J;
or $R_4$ and $R_5$ may be taken together along with the $-(O)_m-P(=O)-(O)_n-$ of Q to form a 5-8 membered heterocyclic ring optionally substituted with J;
or $R_4$ and $R_5$ may be taken together to form an aralkyl group;

with the proviso that $R_1$ cannot be methylene substituted with carboxy.

**2.** A compound of claim 1 having the formula:

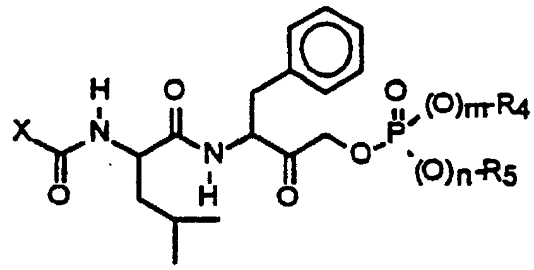

3. A compound of claim 2 wherein $R_4$ and $R_5$ are independently selected from the group consisting of hydrogen; alkyl having from 1 to 4 carbons, alkenyl and alkynyl each having up to 4 carbons and one or two sites of unsaturation, cycloalkyl having up to 4 carbons, said alkyl, alkenyl, alkynyl and cycloalkyl groups optionally substituted with J; aryl substituted with J; and aralkyl optionally substituted with J;

or $R_4$ and $R_5$ taken together along with the $-(O)_m-P(=O)-(O)_n-$ of Q form a six membered ring that is substituted by J.

4. A compound of claim 3 wherein J is independently selected from the group consisting of alkyl having 1 to 10 carbons, alkenyl and alkynyl each having up to 10 carbons and one or two sites of unsaturation, cycloalkyl having up to 10 carbons; aryl; aryloxy; and halogen.

5. A compound of claim 4 wherein $R_4$ and $R_5$ are independently selected from the group consisting of hydrogen; alkyl having from 1 to 4 carbons, alkenyl and alkynyl having up to 4 carbons and one or two sites of unsaturation, cycloalkyl having up to 4 Carbons, said alkyl, alkenyl, alkynyl and cycloalkyl groups optionally substituted with any of alkyl having from 1 to 10 carbons, alkenyl and alkynyl each having up to 10 carbons and one or two sites of unsaturation, cycloalkyl having up to 10 carbons and aryl; aryl substituted with halogen; aralkyl; aralkyl substituted with alkyl; and aralkyl substituted with aryloxy;

or $R_4$ and $R_5$ taken together along with the $-(O)_m-P(=O)-(O)_n-$ of Q form a six membered ring that is substituted by aralkyloxy.

6. A compound of claim 5 wherein $R_4$ and $R_5$ are independently selected from the group consisting of H; methyl; butyl; 2-ethylhexyl; 2-cyclohexylethyl; 2-phenylethyl; 4-chlorophenyl; benzyl; 2-methylbenzyl; and 3-phenoxybenzyl;

or $R_4$ and $R_5$ taken together along with the $-(O)_m-P(=O)-(O)_n-$ of Q form a six-membered ring having the formula:

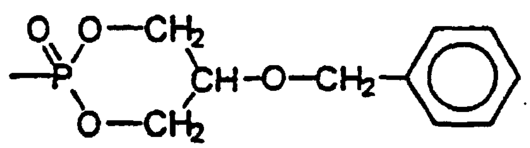

7. A compound of claim 6 wherein $R_4$ and $R_5$ are independently selected from the group consisting of benzyl; 2-methylbenzyl; and 2-phenylethyl.

8. A compound of claim 2 wherein X is selected from the group consisting of alkoxy having 1 to 10 carbons; aralkyloxy having from 7 to 15 carbons; and a carbohydrate moiety optionally containing one or more alkylated hydroxyl groups.

9. A compound of claim 8 wherein X is selected from the group consisting of benzyloxy; t-butoxy; diisopropylidine-2-keto-L-gulonyl; and monoisopropylidine-2-keto-L-gulonyl.

10. A compound of claim 1 wherein J is selected from the group consisting of halogen; alkyl having 1 to 4 carbons, alkenyl and alkynyl having up to 4 carbons and one or two sites of unsaturation, cycloalkyl having up to 4 carbons; aryl; heteroaryl; amino optionally substituted with one to three of any of aryl, alkyl having 1 to 4 carbons, alkenyl

and alkynyl each having up to 4 carbons and one or two sites of unsaturation and cycloalkyl having up to 4 carbons; guanidino; alkoxycarbonyl; alkoxy; hydroxy; and carboxy; and $R_4$ and $R_5$ are each independently selected from the group consisting of hydrogen; alkyl having 1 to 4 carbons, alkenyl and alkynyl each having up to 4 carbons and one or two sites of unsaturation, cycloalkyl having up to 4 carbons, said alkyl, alkenyl, alkynyl and cycloalkyl groups optionally substituted with J; heteroalkyl optionally substituted with J; or $R_4$ and $R_5$ may be taken together along with the $-(O)_m-P(=O)-(O)_n-$ of Q to form a 5-8 membered heterocyclic ring; or $R_4$ and $R_5$ may be taken together to form a 5-8 membered ring optionally substituted with J.

**11.** A compound of claim I wherein X is benzyloxy; W is carbonyl; Y is NH; $R_1$ is benzyl; $R_2$ is isobutyl; $R_3$ is hydrogen; and $R_4$ and $R_5$ are each 2-methylbenzyl.

**12.** A compound of claim 1 wherein X is benzyloxy; W is carbonyl; Y is NH; $R_1$ is benzyl; $R_2$ is isobutyl; $R_3$ is hydrogen; and $R_4$ and $R_5$ are each 2-phenylethyl.

**13.** A compound of claim 1 wherein $R_1$ is $CH_2$ Ph, $R_2$ is iBu, $R_3$ is H and X-W-Y and Q are selected from the group comprising X-W-Y and Q, in combination, as follows:

| X-W-Y | Q |
|---|---|
| $C_6H_5CH_2OCONH$ | $OP(O)(OCH_2C_6H_5)_2$ |
| t-$C_4H_9OCONH$ | $OP(O)(OCH_2C_6H_5)_2$ |
| Morpholinyl sulfonyl-NH | $OP(O)(OCH_2C_6H_5)_2$ |
| Diisopropylidine-2-keto-L-gulonyl-NH | $OP(O)(OCH_2C_6H_5)_2$ |
| Monoisopropylidine-2-keto-L-gulonyl-NH | $OP(O)(OCH_2C_6H_5)_2$ |
| $C_6H_5CH_2OCONH$ | $OP(O)(OCH_2C_6H_4$-2-$CH_3)_2$ |
| $C_6H_5CH_2OCONH$ | $OP(O)(OCH_2C_6H_4$-3-$OC_6H_5)_2$ |
| t-$C_4H_9OCONH$ | $OP(O)(OH)_2$ |
| $C_6H_5CH_2OCONH$ | $OP(O)(OCH_3)_2$ |
| $C_6H_5CH_2OCONH$ | $OP(O)(OC_4H_9)_2$ |
| $C_6H_5CH_2OCONH$ | $OP(O)(OCH_2CH(C_2H_5)C_4H_9)_2$ |
| t-$C_4H_9OCONH$ | $OP(O)OCH_2CH_2$-cyclohexyl$)_2$ |
| $C_6H_5CH_2OCONH$ | $OP(O)(OCH_2CH_2C_6H_5)_2$ |
| $C_6H_5CH_2OCONH$ | $OP(O)(OCH_2)_2CHOCH_2C_6H_5$ |

**14.** A composition for inhibiting a protease selected from the group consisting of serine proteases and cysteine proteases comprising a compound of any one of claims 1 to 13.

**15.** A method for inhibiting a protease comprising contacting *in vitro* a protease selected from the group consisting of serine proteases and cysteine proteases with an inhibitory amount of a compound of any one of claims I to 13.

**16.** A compound of any one of claim 1 to 13 for use in a method for inhibiting a protease selected from the group consisting of serine proteases and cysteine proteases.

**17.** Use of a compound of any one of claims I to 13 in the manufacture of a medicament for inhibiting a protease selected from the group consisting of serine proteases and cystein proteases.

**Patentansprüche**

**1.** Verbindung der Formel

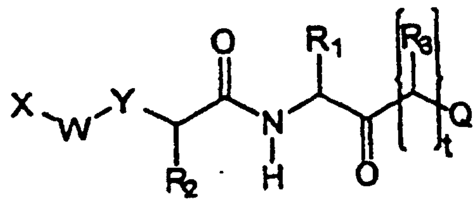

worin
X aus der Gruppe, bestehend aus Aryl mit 6 bis 14 Kohlenstoffatomen, Heteroaryl mit 6 bis 14 Ringatomen, Aralkyl mit 7 bis 15 Kohlenstoffatomen, Alkyl mit 1 bis 10 Kohlenstoffatomen, Alkenyl und Alkinyl mit jeweils bis zu 10 Kohlenstoffatomen und eine oder zwei ungesättigte Stellen enthaltend, Cycloalkyl mit bis zu 10 Kohlenstoffatomen, wobei die Alkyl-, Alkenyl-, Alkinylund Cycloalkylgruppen gegebenenfalls mit einer oder mehreren Gruppen J substituiert sind, Heterocycloalkyl mit 2 bis 7 Kohlenstoffatomen, Alkoxy mit 1 bis 10 Kohlenstoffatomen, Aralkyloxy mit 7 bis 15 Kohlenstoffatomen und einem Kohlenhydratteil, der gegebenenfalls eine oder mehrere alkylierte Hydroxylgruppen enthält, ausgewählt ist,
W aus der Gruppe, bestehend aus Carbonyl und $SO_2$, ausgewählt ist,
Y aus der Gruppe, bestehend aus NH und $(CH_2)_k$, worin k eine ganze Zahl von 0 bis 3 ist, ausgewählt ist,
$R_1$ und $R_2$ unabhängig aus der Gruppe, bestehend aus Wasserstoff, Alkyl mit 1 bis 14 Kohlenstoffatomen, Alkenyl und Alkinyl mit jeweils bis zu 10 Kohlenstoffatomen und eine oder zwei ungesättigte Stellen enthaltend und Cycloalkyl mit 3 bis 10 Kohlenstoffatomen, wobei die Alkyl-, Alkenyl-, Alkinylund Cycloalkylgruppen gegebenenfalls mit einer oder mehreren Gruppen J substituiert sind, ausgewählt sind,
$R^3$ aus der Gruppe, bestehend aus Wasserstoff, Alkyl mit 1 bis 4 Kohlenstoffatomen, Alkenyl und Alkinyl mit jeweils bis zu 4 Kohlenstoffatomen und eine oder zwei ungesättigte Stellen enthaltend, Cycloalkyl mit bis zu 4 Kohlenstoffatomen, Aryl, Heteroaryl, Aralkyl und Heteroaralkyl, ausgewählt ist,
t 1 ist,
J aus der Gruppe, bestehend aus Halogen, Alkyl mit 1 bis 10 Kohlenstoffatomen, Alkenyl und Alkinyl mit jeweils bis zu 10 Kohlenstoffatomen und eine oder zwei ungesättigte Stellen aufweisend, Cycloalkyl mit bis zu 10 Kohlenstoffatomen, Aryl, Heteroaryl, Amino, gegebenenfalls mit einem bis drei irgendeines von Aryl, Alkyl mit 1 bis 4 Kohlenstoffatomen, Alkenyl oder Alkinyl mit jeweis bis zu 4 Kohlenstoffatomen und eine oder zwei ungesättigte Stellen aufweisend und Cycloalkyl mit bis zu 4 Kohlenstoffatomen substituiert, Guanidino, Alkoxycarbonyl, Alkoxy, Hydroxy, Aryloxy, Aralkyloxy, Heterocycloalkyl und Carboxy, ausgewählt ist und
Q die Formel

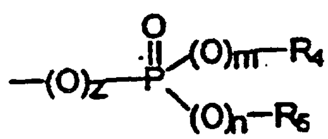

aufweist, worin
m, n und z jeweils 1 sind,
$R_4$ und $R_5$ jeweils unabhängig aus der Gruppe, bestehend aus Wasserstoff, Alkyl mit 1 bis 4 Kohlenstoffatomen, Alkenyl und Alkinyl mit jeweils bis zu 4 Kohlenstoffatomen und eine oder zwei ungesättigte Stellen aufweisend, Cycloalkyl mit bis zu 4 Kohlenstoffatomen, wobei die Alkyl-, Alkenyl-, Alkinylund Cycloalkylgruppen gegebenenfalls mit J substituiert sind, Aryl, gegebenenfalls mit J substituiert, Aralkyl, gegebenenfalls mit J substituiert, und Heteroaryl, gegebenenfalls mit J substituiert, ausgewählt sind,
oder $R_4$ und $R_5$ mit $-(O)_m-P(=O)-(O)_n-$ von Q zusammengefaßt sein können, um einen 5- bis 8-gliedrigen heterocyclischen Ring, gegebenenfalls mit J substituiert, zu bilden,
oder $R_4$ und $R_5$ zusammengefaßt sein können, um eine Aralkylgruppe zu bilden,
mit der Maßgabe, daß $R_1$ nicht mit Carboxy substituiertes Methylen sein kann.

**2.** Verbindung nach Anspruch 1, die Formel

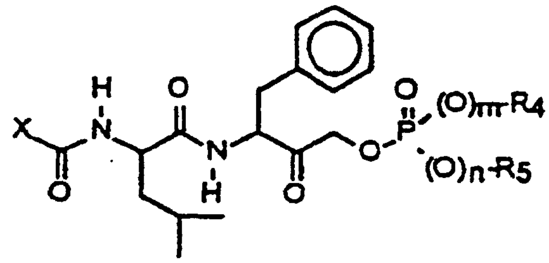

aufweisend.

**3.** Verbindung nach Anspruch 2, wobei $R^4$ und $R^5$ unabhängig aus der Gruppe, bestehend aus Wasserstoff, Alkyl mit 1 bis 4 Kohlenstoffatomen, Alkenyl und Alkinyl mit jeweils bis zu 4 Kohlenstoffatomen und eine oder zwei ungesättigte Stellen aufweisend, Cycloalkyl mit bis zu 4 Kohlenstoffatomen, wobei die Alkyl-, Alkenyl-, Alkinyl- und Cycloalkylgruppen gegebenenfalls mit J substituiert sind, Aryl, mit J substituiert, und Aralkyl, gegebenenfalls mit J substituiert, ausgewählt sind,
oder $R_4$ und $R_5$, mit $-(O)_m-P(=O)-(O)_n-$ von Q zusammengefaßt, einen sechsgliedrigen Ring, der mit J substituiert ist, bilden.

**4.** Verbindung nach Anspruch 3, wobei J unabhängig aus der Gruppe, bestehend aus Alkyl mit 1 bis 10 Kohlenstoffatomen, Alkenyl oder Alkinyl mit jeweils bis zu 10 Kohlenstoffatomen und eine oder zwei ungesättigte Stellen aufweisend, Cycloalkyl mit bis zu 10 Kohlenstoffatomen, Aryl, Aryloxy und Halogen, ausgewählt ist.

**5.** Verbindung nach Anspruch 4, wobei $R_4$ und $R_5$ unabhängig aus der Gruppe, bestehend aus Wasserstoff, Alkyl mit 1 bis 4 Kohlenstoffatomen, Alkenyl oder Alkinyl mit bis zu 4 Kohlenstoffatomen und eine oder zwei ungesättigte Stellen aufweisend, Cycloalkyl mit bis zu 4 Kohlenstoffatomen, wobei die Alkyl-, Alkenyl-, Alkinyl- und Cycloalkylgruppen gegebenenfalls mit jeglichem von Alkyl mit 1 bis 10 Kohlenstoffatomen, Alkenyl und Alkinyl mit jeweils bis zu 10 Kohlenstoffatomen und eine oder zwei ungesättigte Stellen aufweisend, Cycloalkyl mit bis zu 10 Kohlenstoffatomen und Aryl substituiert ist, mit Halogen substituiertes Aryl, Aralkyl, mit Alkyl substituiertes Aralkyl und mit Aryloxy substituiertes Aralkyl, ausgewählt sind,
oder $R_4$ und $R_5$, mit $-(O)_m-P(=O)-(O)_n-$ von Q zusammengefaßt, einen sechsgliedrigen Ring, der mit Aralkyloxy substituiert ist, bilden.

**6.** Verbindung nach Anspruch 5, wobei $R_4$ und $R_5$ unabhängig aus der Gruppe, bestehend aus H, Methyl, Butyl, 2-Ethylhexyl, 2-Cyclohexylethyl, 2-Phenylethyl, 4-Chlorphenyl, Benzyl, 2-Methylbenzyl und 3-Phenoxybenzyl, ausgewählt sind,
oder $R_4$ und $R_5$, mit $-(O)_m-P(=O)-(O)_n-$ von Q zusammengefaßt, einen sechsgliedrigen Ring bilden, die Formel

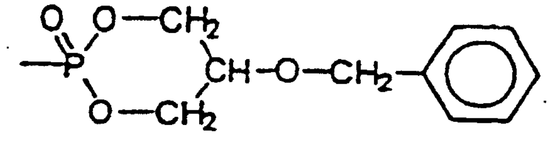

aufweisend.

**7.** Verbindung nach Anspruch 6, wobei $R_4$ und $R_5$ unabhängig aus der Gruppe, bestehend aus Benzyl, 2-Methylbenzyl und 2-Phenylethyl, ausgewählt sind.

**8.** Verbindung nach Anspruch 2, wobei X aus der Gruppe, bestehend aus Alkoxy mit 1 bis 10 Kohlenstoffatomen, Aralkoxy mit 7 bis 15 Kohlenstoffatomen und einem Kohlenhydratteil, der gegebenenfalls eine oder mehrere alkylierte Hydroxylgruppen enthält, ausgewählt ist.

**9.** Verbindung nach Anspruch 8, wobei X aus der Gruppe, bestehend aus Benzyloxy, t-Butoxy, Diisopropylidin-2-keto-L-gulonyl und Monoisopropylidin-2-keto-L-gulonyl, ausgewählt ist.

**10.** Verbindung nach Anspruch 1, wobei J aus der Gruppe, bestehend aus Halogen, Alkyl mit 1 bis 4 Kohlenstoffatomen, Alkenyl und Alkinyl mit bis zu 4 Kohlenstoffatomen und eine oder zwei ungesättigte Stellen aufweisend, Cycloalkyl mit bis zu 4 Kohlenstoffatomen, Aryl, Heteroaryl, Amino, gegebenenfalls mit einem bis drei irgendeines von Aryl, Alkyl mit 1 bis 4 Kohlenstoffatomen, Alkenyl und Alkinyl mit jeweils bis zu 4 Kohlenstoffatomen und eine oder zwei ungesättigte Stellen aufweisend und Cycloalkyl mit bis zu 4 Kohlenstoffatomen substituiert, Guanidino, Alkoxycarbonyl, Alkoxy, Hydroxy und Carboxy, ausgewählt ist; und $R_4$ und $R_5$ jeweils unabhängig aus der Gruppe, bestehend aus Wasserstoff, Alkyl mit 1 bis 4 Kohlenstoffatomen, Alkenyl und Alkinyl mit jeweils bis zu 4 Kohlenstoffatomen und eine oder zwei ungesättigte Stellen aufweisend, Cycloalkyl mit bis zu 4 Kohlenstoffatomen, wobei die Alkyl-, Alkenyl-, Alkinyl- und Cycloalkylgruppen gegebenenfalls mit J substituiert sind, Heteroalkyl, gegebenenfalls mit J substituiert, ausgewählt sind, oder $R_4$ und $R_5$, mit $-(O)_m-P(=O)-(O)_n-$ von Q zusammengefaßt sein können, um einen 5- bis 8-gliedrigen heterocyclischen Ring zu bilden, oder $R_4$ und $R_5$ zusammengefaßt sein können, um einen 5- bis 8-gliedrigen Ring zu bilden, der gegebenenfalls mit J substituiert ist.

**11.** Verbindung nach Anspruch 1, wobei X Benzyloxy ist, W Carbonyl ist, Y NH ist, $R_1$ Benzyl ist, $R_2$ Isobutyl ist, $R_3$ Wasserstoff ist und $R_4$ und $R_5$ jeweils 2-Methylbenzyl sind.

**12.** Verbindung nach Anspruch 1, wobei X Benzyloxy ist, W Carbonyl ist, Y NH ist, $R_1$ Benzyl ist, $R_2$ Isobutyl ist, $R_3$ Wasserstoff ist und $R_4$ und $R_5$ jeweils 2-Phenylethyl sind.

**13.** Verbindung nach Anspruch 1, wobei $R_1$ $CH_2Ph$ ist, $R_2$ iBu ist, $R_3$ H ist und X-W-Y und Q aus der Gruppe, umfassend X-W-Y und Q, in Kombination, wie folgt, ausgewählt sind:

| X-W-Y | Q |
|---|---|
| $C_6H_5CH_2OCONH$ | $OP(O)(OCH_2C_6H_5)_2$ |
| t-$C_4H_9OCONH$ | $OP(O)(OCH_2C_6H_5)_2$ |
| Morpholinyl sulfonyl-NH | $OP(O)(OCH_2C_6H_5)_2$ |
| Diisopropylidin -2-keto-L-gulonyl-NH | $OP(O)(OCH_2C_6H_5)_2$ |
| Monoisopropylidin -2-keto-L-gulonyl-NH | $OP(O)(OCH_2C_6H_5)_2$ |
| $C_6H_5CH_2OCONH$ | $OP(O)(OCH_2C_6H_4$-2-$CH_3)_2$ |
| $C_6H_5CH_2OCONH$ | $OP(O)(OCH_2C_6H_4$-3-$OC_6H_5)_2$ |
| t-$C_4H_9OCONH$ | $OP(O)(OH)_2$ |
| $C_6H_5CH_2OCONH$ | $OP(O)(OCH_3)_2$ |
| $C_6H_5CH_2OCONH$ | $OP(O)(OC_4H_9)_2$ |
| $C_6H_5CH_2OCONH$ | $OP(O)(OCH_2CH(C_2H_5)C_4H_9)_2$ |
| t-$C_4H_9OCONH$ | $OP(O)OCH_2CH_2$-Cyclohexyl$)_2$ |
| $C_6H_5CH_2OCONH$ | $OP(O)(OCH_2CH_2C_6H_5)_2$ |
| $C_6H_5CH_2OCONH$ | $OP(O)(OCH_2)_2CHOCH_2C_6H_5$ |

**14.** Zusammensetzung zur Hemmung einer Protease, ausgewählt aus der Gruppe, bestehend aus Serinproteasen und Cysteinproteasen, umfassend eine Verbindung nach einem der Ansprüche 1 bis 13.

**15.** Verfahren zur Hemmung einer Protease, umfassend das Kontaktieren *in vi*tro einer Protease, ausgewählt aus der Gruppe, bestehend aus Serinproteasen und Cysteinproteasen, mit einer hemmenden Menge einer Verbindung nach einem der Ansprüche 1 bis 13.

**16.** Verbindung nach einem der Ansprüche 1 bis 13 zur Verwendung in einem Verfahren zur Hemmung einer Protease, ausgewählt aus der Gruppe, bestehend aus Serinproteasen und Cysteinproteasen.

**17.** Verwendung einer Verbindung nach einem der Ansprüche 1 bis 13 zur Herstellung eines Medikaments zur Hemmung einer Protease, ausgewählt aus der Gruppe, bestehend aus Serinproteasen und Cysteinproteasen.

**Revendications**

**1.** Composé de formule :

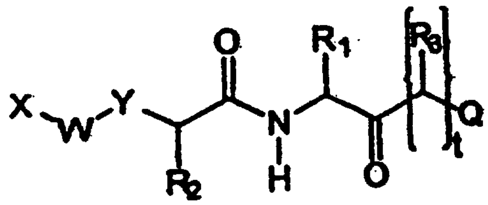

dans laquelle :

X est choisi dans le groupe constitué par un groupement aryle ayant de 6 à 14 atomes de carbone ; un groupement hétéroaryle ayant de 6 à 14 atomes dans le cycle ; un groupement aralkyle ayant de 7 à 15 atomes de carbone ; un groupement alkyle ayant de 1 à 10 atomes de carbone ; un groupement alcényle et alcynyle ayant chacun jusqu'à 10 atomes de carbone et contenant un ou deux sites d'insaturation ; un groupement cycloalkyle ayant jusqu'à 10 atomes de carbone, lesdits groupements alkyle, alcényle, alcynyle et cycloalkyle étant facultativement substitués par un ou plusieurs groupements J ; un groupement hétérocycloalkyle ayant de 2 à 7 atomes de carbone ; un groupement alcoxy ayant de 1 à 10 atomes de carbone ; un groupement aralkyloxy ayant de 7 à 15 atomes de carbone ; et un groupement glucidique contenant facultativement un ou plusieurs groupements hydroxyle alkylés ;
W est choisi dans le groupe constitué par un carbonyle et $SO_2$ ;
Y est choisi dans le groupe constitué par NH et $(CH_2)_k$ dans lequel k est un nombre entier de 0 à 3 ;
$R_1$ et $R_2$ sont choisi indépendamment dans le groupe constitué par un hydrogène ; un groupement alkyle ayant de 1 à 14 atomes de carbone ; un groupement alcényle et alcynyle ayant chacun jusqu'à 10 atomes de carbone et contenant un ou deux sites d'insaturation et un groupement cycloalkyle ayant de 3 à 10 atomes de carbone, lesdits groupements alkyle, alcényle, alcynyle et cycloalkyle étant facultativement substitués par un ou plusieurs groupements J ;
$R_3$ est choisi dans le groupe constitué par un hydrogène ; un groupement alkyle ayant de 1 à 4 atomes de carbone ; un groupement alcényle et alcynyle ayant chacun jusqu'à 4 atomes de carbone et contenant un ou deux sites d'insaturation ; un groupement cycloalkyle ayant jusqu'à 4 atomes de carbone ; un groupement aryle ; un groupement hétéroaryle ; un groupement aralkyle et un groupement hétéroaralkyle ; t vaut 1 ;
J est choisi dans le groupe constitué par un halogène ; un groupement alkyle ayant de 1 à 10 atomes de carbone ; un groupement alcényle et alcynyle ayant chacun jusqu'à 10 atomes de carbone et un ou deux sites d'insaturation ; un groupement cycloalkyle ayant jusqu'à 10 atomes de carbone ; un groupement aryle ; un groupement hétéroaryle ; un groupement amino facultativement substitué par 1 à 3 de l'un quelconque d'un groupement aryle, d'un groupement alkyle ayant de 1 à 4 atomes de carbone, d'un groupement alcényle ou alcynyle ayant chacun jusqu'à 4 atomes de carbone et un ou deux sites d'insaturation et d'un groupement cycloalkyle ayant jusqu'à 4 atomes de carbone ; un groupement guanidino ; un groupement alcoxycarbonyle ; un groupement alcoxy, un groupement hydroxy ; un groupement aryloxy ; un groupement aralkyloxy ; un groupement hétérocycloalkyle et un groupement carboxy ; et
Q a la formule :

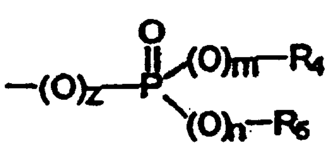

dans laquelle :

m, n et z valent chacun 1 ;
$R_4$ et $R_5$ sont chacun indépendamment choisis parmi le groupe constitué par un hydrogène ; un groupement alkyle ayant de 1 à 4 atomes de carbone ; un groupement alcényle et alcynyle ayant chacun jusqu'à 4 atomes de carbone et un ou deux sites d'insaturation et un groupement cycloalkyle ayant jusqu'à 4 atomes de carbone, lesdits groupements alkyle, alcényle, alcynyle et cycloalkyle étant facultativement substitués par le groupement J ; un groupement aryle facultativement substitué par le groupement J ; un groupement aralkyle facultativement substitué par le groupement J et un groupement hétéroaryle facultativement substitué par le groupement J ;
ou $R_4$ et $R_5$ peuvent être pris ensembles avec le groupement $-(O)_m-P(=O)-(O)_n-$ de Q, pour former un cycle hétérocyclique à 5 à 8 membres, facultativement substitué par le groupement J ;
ou $R_4$ et $R_5$ peuvent être pris ensembles pour former un groupement aralkyle ;
pourvu que $R_1$ ne puisse pas être un méthylène substitué par un groupement carboxy.

2. Composé selon la revendication 1, ayant la formule :

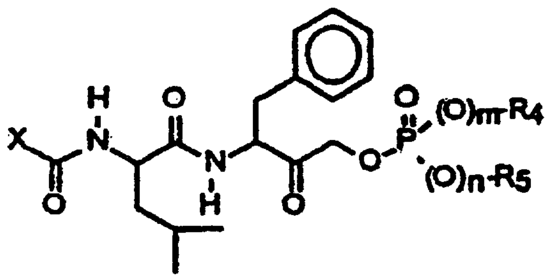

3. Composé selon la revendication 2, dans lequel $R_4$ et $R_5$ sont indépendamment choisis dans le groupe constitué par un hydrogène ; un groupement alkyle ayant de 1 à 4 atomes de carbone ; un groupement alcényle et alcynyle ayant chacun jusqu'à 4 atomes de carbone et un ou deux sites d'insaturation ; un groupement cycloalkyle ayant jusqu'à 4 atomes de carbone, lesdits groupements alkyle, alcényle, alcynyle et cycloalkyle étant facultativement substitués par le groupement J ; un groupement aryle substitué par le groupement J et un groupement aralkyle facultativement substitué par le groupement J ;
ou $R_4$ et $R_5$ pris ensembles avec le groupement $-(O)_m-P(=O)-(O)_n-$ de Q, forment un cycle à six membres, qui est substitué par le groupement J.

4. Composé selon la revendication 3, dans lequel J est indépendamment choisi dans le groupe constitué par un groupement alkyle ayant de 1 à 10 atomes de carbone ; un groupement alcényle et alcynyle ayant chacun jusqu'à 10 atomes de carbone et un ou deux sites d'insaturation ; un groupement cycloalkyle ayant jusqu'à 10 atomes de carbone ; un groupement aryle ; un groupement aryloxy et un halogène.

5. Composé selon la revendication 4, dans lequel $R_4$ et $R_5$ sont indépendamment choisis dans le groupe constitué par un hydrogène ; un groupement alkyle ayant de 1 à 4 atomes de carbone ; un groupement alcényle et alcynyle ayant chacun jusqu'à 4 atomes de carbone et un ou deux sites d'insaturation ; un groupement cycloalkyle ayant jusqu'à 4 atomes de carbone, lesdits groupements alkyle, alcényle, alcynyle et cycloalkyle étant facultativement substitués par l'un quelconque d'un groupement alkyle ayant de 1 à 10 atomes de carbone, d'un groupement alcényle et alcynyle ayant chacun jusqu'à 10 atomes de carbone et un ou deux sites d'insaturation ; d'un groupement cycloalkyle ayant jusqu'à 10 atomes de carbone et d'un groupement aryle ; un groupement aryle substitué par un halogène ; un groupement aralkyle ; un groupement aralkyle substitué par un groupement alkyle ; un groupement aralkyle substitué par un groupement aryloxy ;
ou $R_4$ et $R_5$ pris ensembles avec le groupement $-(O)_m-P(=O)-(O)^n-$ de Q, forment un cycle à 6 membres, qui est substitué par un groupement aralkyloxy.

6. Composé selon la revendication 5, dans lequel $R_4$ et $R_5$ sont indépendamment choisis dans le groupe constitué par H ; le méthyle ; le butyle ; le 2-éthylhexyle ; le 2-cyclohexyléthyle ; le 2-phényléthyle ; le 4-chlorophényle ; le benzyle ; le 2-méthylbenzyle et le 3-phénoxybenzyle ;
ou $R_4$ et $R_5$ pris ensembles avec le groupement $-(O)_m-P(=O)-(O)_n-$ de Q, forment un cycle à 6 membres ayant la formule :

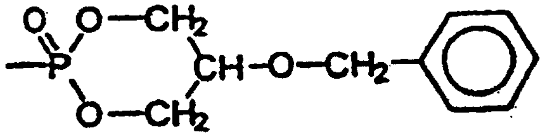

7. Composé selon la revendication 6, dans lequel $R_4$ et $R_5$ sont indépendamment choisis dans le groupe constitué par le benzyle ; le 2-méthylbenzyle et le 2-phényléthyle.

8. Composé selon la revendication 2, dans lequel X est choisi dans le groupe constitué par un groupement alcoxy ayant de 1 à 10 atomes de carbone ; un groupement aralkyloxy ayant de 7 à 15 atomes de carbone et un groupement glucidique contenant facultativement un ou plusieurs groupements hydroxyle alkylés.

9. Composé selon la revendication 8, dans lequel X est choisi dans le groupe constitué par le benzyloxy ; le t-butoxy ; le diisopropylidin-2-céto-L-gulonyle et le monoisopropylidin-2-céto-L-gulonyle.

10. Composé selon la revendication 1, dans lequel J est choisi dans le groupe constitué par un halogène ; un groupement alkyle ayant de 1 à 4 atomes de carbone ; un groupement alcényle et alcynyle ayant jusqu'à 4 atomes de carbone et un ou deux sites d'insaturation ; un groupement cycloalkyle ayant jusqu'à 4 atomes de carbone ; un groupement aryle ; un groupement hétéroaryle ; un groupement amino facultativement substitué par 1 à 3 de l'un quelconque d'un groupement aryle, d'un groupement alkyle ayant de 1 à 4 atomes de carbone, d'un groupement alcényle et alcynyle ayant chacun jusqu'à 4 atomes de carbone et un ou deux sites d'insaturation et d'un groupement cycloalkyle ayant jusqu'à 4 atomes de carbone ; un groupement guanidino ; un groupement alcoxycarbonyle ; un groupement alcoxy, un groupement hydroxy et un groupement carboxy ; et $R_4$ et $R_5$ sont chacun indépendamment choisis dans le groupe constitué par un hydrogène ; un groupement alkyle ayant de 1 à 4 atomes de carbone ; un groupement alcényle et alcynyle ayant chacun jusqu'à 4 atomes de carbone et un ou deux sites d'insaturation, un groupement cycloalkyle ayant jusqu'à 4 atomes de carbone, lesdits groupements alkyle, alcényle, alcynyle et cycloalkyle étant facultativement substitués par le groupement J ; un groupement hétéroalkyle facultativement substitué par le groupement J ; ou $R_4$ et $R_5$ peuvent être pris ensembles avec le groupement $-(O)_m-P(=O)-(O)_n-$ de Q, pour former un cycle hétérocyclique à 5 à 8 membres ; ou $R_4$ et $R_5$ peuvent être pris ensembles pour former un cycle à 5 à 8 membres facultativement substitué par le groupement J.

11. Composé selon la revendication 1, dans lequel X est un benzyloxy ; W est un carbonyle ; Y est NH ; $R_1$ est un benzyle ; $R_2$ est un isobutyle ; $R_3$ est un hydrogène et $R_4$ et $R_5$ sont chacun un 2-méthylbenzyle.

12. Composé selon la revendication 1, dans lequel X est un benzyloxy ; W est un carbonyle ; Y est NH ; $R_1$ est un benzyle ; $R_2$ est un isobutyle ; $R_3$ est un hydrogène et $R_4$ et $R_5$ sont chacun un 2-phényléthyle.

13. Composé selon la revendication 1, dans lequel $R_1$ est le $CH_2Ph$, $R_2$ est un iBu, $R_3$ est un H et X-W-Y et Q sont choisis dans le groupe comprenant X-W-Y et Q, en combinaison, de la façon suivante :

| X-W-Y | Q |
|---|---|
| $C_6H_5CH_2OCONH$ | $OP(O)(OCH_2C_6H_5)_2$ |
| $t-C_4H_9OCONH$ | $OP(O)(OCH_2C_6H_5)_2$ |
| Morpholinylsulfonyl-NH | $OP(O)(OCH_2C_6H_5)_2$ |
| Diisopropylidin-2-céto-L-gulonyl-NH | $OP(O)(OCH_2C_6H_5)_2$ |
| Monoisopropylidin-2-céto-L-gulonyl-NH | $OP(O)(OCH_2C_6H_5)_2$ |
| $C_6H_5CH_2OCONH$ | $OP(O)(OCH_2C_6H_4-2-CH_3)_2$ |
| $C_6H_5CH_2OCONH$ | $OP(O)(OCH_2C_6H_4-3-OC_6H_5)_2$ |
| $t-C_4H_9OCONH$ | $OP(O)(OH)_2$ |
| $C_6H_5CH_2OCONH$ | $OP(O)(OCH_3)_2$ |
| $C_6H_5CH_2OCONH$ | $OP(O)(OC_4H_9)_2$ |

(suite)

| X-W-Y | Q |
|---|---|
| $C_6H_5CH_2OCONH$ | $OP(O)(OCH_2CH(C_2H_5)C_4H_9)_2$ |
| t-$C_4H_9OCONH$ | OP(O) ($OCH_2CH_2$-cyclohexyle)$_2$ |
| $C_6H_5CH_2OCONH$ | $OP(O)(OCH_2CH_2C_6H_5)_2$ |
| $C_6H_5CH_2OCONH$ | $OP(O)(OCH_2)_2CHOCH_2C_6H_5$ |

**14.** Composition pour inhiber une protéase choisie dans le groupe constitué par les protéases à sérine et les protéases à cystéine, comprenant un composé selon l'une quelconque des revendications 1 à 13.

**15.** Procédé pour inhiber une protéase, comprenant la mise en contact *in vitro* d'une protéase choisie dans le groupe constitué par les protéases à sérine et les protéases à cystéine, avec une quantité inhibitrice d'un composé selon l'une quelconque des revendications 1 à 13.

**16.** Composé selon l'une quelconque des revendications 1 à 13, à utiliser dans un procédé pour inhiber une protéase choisie dans le groupe constitué par les protéases à sérine et les protéases à cystéine.

**17.** Utilisation d'un composé selon l'une quelconque des revendications 1 à 13, dans la fabrication d'un médicament pour inhiber une protéase choisie dans le groupe constitué par les protéases à sérine et les protéases à cystéine.